(19) Europäisches Patentamt

European Patent Office

Office européen des brevets

(11) **EP 1 292 824 B1**

(12) **EUROPÄISCHE PATENTSCHRIFT**

(45) Veröffentlichungstag und Bekanntmachung des Hinweises auf die Patenterteilung:
**24.08.2005 Patentblatt 2005/34**

(21) Anmeldenummer: **01955316.3**

(22) Anmeldetag: **19.06.2001**

(51) Int Cl.7: **G01N 33/38**, G01N 27/02

(86) Internationale Anmeldenummer:
**PCT/EP2001/006898**

(87) Internationale Veröffentlichungsnummer:
**WO 2001/098777 (27.12.2001 Gazette 2001/52)**

(54) **VERFAHREN UND VORRICHTUNG ZUR BESTIMMUNG MINDESTENS EINES PARAMETERS EINER MISCHUNGSFORMEL EINES GEMISCHES UMFASSEND EINEN TRÄGERSTOFF, WASSER UND GAS**

METHOD AND DEVICE FOR THE DETERMINATION OF AT LEAST ONE PARAMETER OF A MIXTURE OF A SUPPORT, WATER AND GAS

PROCEDE ET DISPOSITIF DE DEFINITION D'AU MOINS UN PARAMETRE D'UN MELANGE D'UN SUPPORT, D'EAU ET DE GAZ

(84) Benannte Vertragsstaaten:
**AT BE CH CY DE DK ES FI FR GB GR IE IT LI LU MC NL PT SE TR**

(30) Priorität: **21.06.2000 DE 10030602**

(43) Veröffentlichungstag der Anmeldung:
**19.03.2003 Patentblatt 2003/12**

(73) Patentinhaber: **Mouhasseb, Haissam**
**8118 Pfaffhausen (CH)**

(72) Erfinder: **Mouhasseb, Haissam**
**8118 Pfaffhausen (CH)**

(74) Vertreter: **Blum, Rudolf Emil**
**c/o E. Blum & Co**
**Patentanwälte VSP**
**Vorderberg 11**
**8044 Zürich (CH)**

(56) Entgegenhaltungen:
DE-C- 19 652 679          GB-A- 2 313 443
US-A- 5 418 466

- **KIRAN POKKULURI: "Effects of admixtures, chlorides, and moisture on dielectric properties of Portland Cement Concrete in the low microwave frequency range" [Online] 31. Oktober 1998 (1998-10-31) XP002180181 Gefunden im Internet: <URL: http://scholar.lib.vt.edu/theses/available /etd-92598-152639/unrestricted/KIRAN2.PDF&gt ; [gefunden am 2001-10-15] Seite 25, Absatz 2 -Seite 26, Absatz 4; Abbildung 3.5**
- **NASSAR E M ET AL: "A PROBE ANTENNA FOR IN SITU MEASUREMENT OF THE COMPLEX DIELECTRIC CONSTANT OF MATERIALS" IEEE TRANSACTIONS ON ANTENNAS AND PROPAGATION, IEEE INC. NEW YORK, US, Bd. 47, Nr. 6, Juni 1999 (1999-06), Seiten 1085-1092, XP000845957 ISSN: 0018-926X**

**Beschreibung**

Hinweis auf verwandte Anmeldungen

Diese Anmeldung beansprucht die Priorität der deutschen Patentanmeldung 100 30 602.0, die am 21. Juni 2000 eingereicht wurde und deren ganze Offenbarung hiermit durch Bezug aufgenommen wird.

Hintergrund

Die Erfindung betrifft ein Verfahren und eine Vorrichtung gemäss Oberbegriff der unabhängigen Ansprüche. Dabei ist mindestens ein Parameter eines Gemisches zu bestimmen, dessen Komponenten einen Trägerstoff, Wasser und Gas umfassen.

Ein Verfahren dieser Art kann insbesondere zur Ermittlung des Feuchtezustands von Betonbauteilen verwendet werden. Die Kenntnis des Feuchtezustands von Betonbauteilen ist häufig für die Vermeidung oder Beurteilung von Schäden, aber auch für die Durchführung von Bau- und Instandsetzungsmassnahmen unverzichtbar. Darüber hinaus muss die Feuchtesituation z. B. beim Verlegen von Böden (Fliesen, Parkett, etc.) auf Estrichen, bei Beschichtungsmassnahmen an Betonoberflächen und bei der Beurteilung der Korrosion an Bewehrungsstählen bekannt sein. Daher kommt der Feuchtemessung insbesondere auch im Betonbau eine hohe Bedeutung zu.

Verfahren dieser Art sind jedoch auch in anderen Bereichen, so z.B. in der Charakterisierung von Pharmazeutika und Lebensmitteln anwendbar, und überall dort, wo mindestens ein Parameter eines Gemisches aus Trägerstoff, Wasser und Gas ermittelt werden muss. Dabei kann der Trägerstoff fest oder flüssig sein.

Um den Aufwand und die Kosten klein zu halten, sollte ein entsprechendes Verfahren möglichst zerstörungsfrei durchgeführt werden können.

Ein Verfahren und eine Vorrichtung zur Ermittlung des Feuchtegehalts in porösen Baustoffen sind aus der Patentschrift DE 196 52 679 C1 vorbekannt. Dort wird die Feuchte des Gemisches durch Beaufschlagung eines Sensors mit elektromagnetischen Wellen mehrerer Frequenzen und Bestimmung des frequenzabhängigen Permittivitätswerts des Gemisches mit Hilfe von sensorspezifisch erstellten Kalibrierungsdaten ermittelt. Anhand eines Gleichungssystems, dem die Mischungsformel von Polder-van Santen/de Loor zugrunde gelegt ist und das nach den frequenzunabhängigen Parametern aufgelöst wird, kann auf den Volumenanteil des Flüssigwassers geschlossen werden.

Es zeigt sich jedoch, dass die Genauigkeit dieses Verfahrens nur beschränkt ist.

Darstellung der Erfindung

Es stellt sich deshalb die Aufgabe, ein Verfahren und eine Vorrichtung der eingangs genannten Art bereitzustellen, die Messungen einer höheren Genauigkeit erlauben.

Erfindungsgemäss wird eine Formel der folgenden Form verwendet:

$$\varepsilon_m = \varepsilon_b + \sum_{i=1}^{n} \frac{v_i}{3} \cdot (\varepsilon_i - \varepsilon_b) \cdot \sum_{j=1}^{3} \frac{\varepsilon_m}{\varepsilon_m + N_{ij} \cdot (\varepsilon_i - \varepsilon_m)}$$

mit $\varepsilon_1$ und $v_1$ Permittivitätswert und Volumenanteil des Gases, $\varepsilon_2$ und $v_2$ Permittivitätswert und Volumenanteil des freien Wassers, $\varepsilon_3$ und $v_3$ Permittivitätswert und Volumenanteil des gebundenen Wassers (sofern dieses berücksichtigt wird), $\varepsilon_b$ Permittivitätswert des Trägerstoffes und $N_{1j}$, $N_{2j}$ und $N_{3j}$ die Depolarisierungsfaktoren einer ellipsoiden Kavität des Gases bzw. des freien Wassers bzw. des gebundenen Wassers sind. Dabei wird $n = 3$ gesetzt bei Berücksichtigung des gebundenen Wassers und $n = 2$ bei Vernachlässigung des gebundenen Wassers.

Wird diese Mischungsformel bei ausreichend vielen Frequenzen ausgemessen, so entsteht ein ausreichend bestimmtes oder überbestimmtes Gleichungssystem, welches erlaubt, mindestens einen der unbekannten Parameter, z.B. den Volumananteil des freien Wassers, zu ermitteln.

In einer bevorzugten Ausführung wird der Permittivitätswert des gebundenen Wassers als frequenzabhängige Funktion in die Mischungsformel eingesetzt. Es zeigt sich, dass durch diese Massnahme eine realistischere Modellierung des Systems erreicht und die Messgenauigkeit verbessert wird.

In einer weiteren bevorzugten Ausführung wird angenommen, dass der Beitrag des gebundenen Wassers nicht separat berücksichtigt werden muss. In diesem Falle werden aus dem Gleichungssysstem die folgenden Parameter (oder von diesen Parametern abgeleitete Grössen) gleichzeitig ermittelt:

-    Volumenanteil $v_1$ des Gases,

- Volumenanteil $v_2$ des freien Wassers,
- mindestens einen der Depolarisierungsfaktoren $N_{2j}$ des freien Wassers und
- Leitfähigkeit des freien Wassers.

Mit anderen Worten werden diese Parameter also alle an die gemessenen Werte angepasst, z.B. durch Ausgleichsrechnung, was eine bessere Modulierung des Systems und somit eine erhöhte Messgenauigkeit ergibt.

In einer weiteren bevorzugten Ausführung der Erfindung wird berücksichtigt, dass der Wasseranteil des Gemisches, oder ein anderer zu bestimmender Parameter, von der Tiefe, d.h. dem Abstand zur Gemisch-Oberfläche variieren kann. Um dies zu berücksichtigen, werden mehrere Messschritte durchgeführt, bei welchen der Sensor in bekanntem Abstand vom Gemisch angeordnet und von diesem durch ein Dielektrikum mit bekannter Permittivität getrennt wird. Bei jedem Messschritt ermittelt der Sensor einen vom integralen Permittivitätswert $\varepsilon_k$ des Gemisches im Messbereich abhängigen Wert $w_k$. Sodann wird eine Auswertung durchgeführt, in welcher über die unterschiedliche Abhängigkeit der Werte $w_k$ vom zu messenden Parameter ein tiefenabhängiger Verlauf des Flüssigwasseranteils ermittelt wird.

## Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:

Fig. 1 einen schematischen Aufbau einer bevorzugten Ausführung der Vorrichtung,
Fig. 2 einen Schnitt durch einen Oberflächensensor und
Fig. 3 einen Schnitt durch einen Hohlleitersensor.

## Wege zur Ausführung der Erfindung

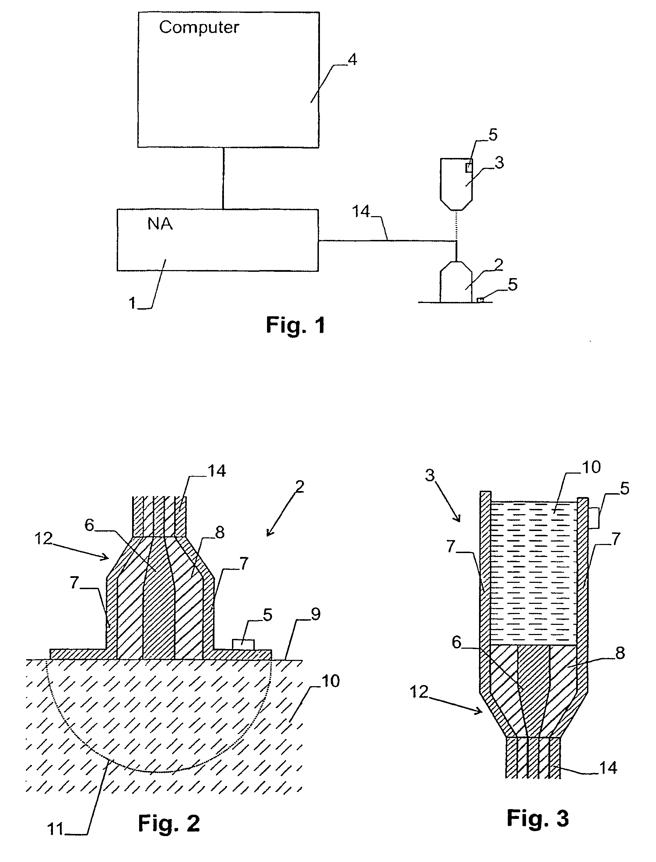

Die Anordnung gemäss Fig. 1 umfasst ein Permittivitätswert-Messgerät 1 zur frequenzabhängigen Bestimmung des komplexen Permittivitätswerts eines Gemisches. Weiter besitzt sie einen Oberflächensensor 2 für die Ausmessung fester Gemische, der auf eine glatte und ebene Fläche aufgesetzt werden kann, und einen Rundhohlleitersensor 3 für die Ausmessung flüssiger Gemische, welche in den Sensor 3 eingefüllt werden. Die Sensoren 2 oder 3 werden wahlweise über eine Koaxialleitung 14 mit dem Permittivitätswert-Messgerät 1 elektrisch verbunden. Das Permittivitätswert-Messgerät 1 besitzt einen vektoriellen Netzwerkanalysator, der den Real- und Imaginärteil des Reflexionsfaktors des jeweiligen Sensors misst. Dieser Reflexionsfaktor wird mittels sensorspezifischer Kalibrierungsdaten in eine komplexe Permittivität $\varepsilon_m$ des Gemisches umgewandelt. Entsprechende Verfahren sind dem Fachmann bekannt.

Zur Auswertung der Messungen ist ein Datenverarbeitungssystem 4, beispielsweise ein herkömmlicher PC, mit dem Permittivitätswert-Messgerät 1 verbunden und steuert den gesamten Mess- und Rechenprozess in der im Folgenden beschriebenen Weise. Ein Temperaturfühler 5 dient zu Erfassung der Temperatur des zu prüfenden Gemisches.

Fig. 2 zeigt einen Schnitt durch den Oberflächensensor 2. Er besitzt einen rotationssysmmetrischen Aufbau mit einem Innenleiter 6 und einem Aussenleiter 7, die durch eine koaxiale Isolierschicht 8, vorzugsweise aus Teflon, voneinander getrennt sind. An seinem Messende ist der Temperatursensor 5 angeordnet.

Der Oberflächensensor 2 kann auf die glatte, ebene Oberfläche 9 des zu messenden Gemisches 10 aufgesetzt werden, so dass sich sein Messbereich 11 in das Gemisch hinein erstreckt. Wie weiter unten ausgeführt, kann er auch im Abstand vom zu messenden Gemisch 10 angeordnet werden, so dass sich sein Messbereich 11 nur teilweise in das Gemisch hinein erstreckt.

Zur Koaxialleitung 14 hin besitzt der Oberflächensensor 2 einen sich verjüngenden Übergangsteil 12. Dieser gewährt einen impedanzangepassten Anschluss des Oberflächensensors 2 an die Koaxialleitung 14. Der Übergang wird von zwei Kegeln mit gemeinsamer Spitze gebildet. Bei einer solchen Geometrie sind Impedanz und Kegelwinkel über folgende Relation verbunden:

$$Z_0 = \frac{Z_{F0}}{2\,\pi\,\sqrt{\varepsilon_c}}\,\ln\frac{\tan\,(\vartheta_2\,/\,2)}{\tan\,(\vartheta_1\,/\,2)} \qquad (1)$$

Dabei ist $Z_0$ die Impedanz des Übergangsteils (die jener des Koxialkabels entsprechen und z.B. 50 Ohm betragen sollte), $Z_{F0}$ die Vakuum-Impedanz ($Z_{F0} = \sqrt{\mu_0/\varepsilon_0} = 377$ Ohm) , $\varepsilon_c$ die Permittivität der Isolierschicht 8, $\vartheta_1$ der Winkel des Innenkegels des Innenleiters 6 und $\vartheta_2$ der Winkel des Aussenkegels des Aussenleiters 7.

Fig. 3 zeigt einen Schnitt durch den Hohlleitersensor 3. Dieser besitzt wiederum einen rotationssysmmetrischen Aufbau mit einem Innenleiter 6 und einem Aussenleiter 7, die durch eine koaxiale Isolierschicht 8, vorzugsweise aus Teflon, voneinander getrennt sind. Der Aussenleiter 7 ragt über die Isolierschicht und den Innenleiter hinaus und begrenzt einen Innenraum zur Aufnahme des zu messenden Gemisches 10. Wiederum kann ein Übergangsteil 12 zur impedanzangepassten Verbindung mit der Koaxialleitung 14 vorgesehen sein.

Mit den Sensoren gemäss Fig. 2 und 3 wird die Permittivität über die Reflexion einer elektromagnetischen Welle bestimmt. Die Permittivität kann jedoch auch aus einer Transmissionsmessung ermittelt werden, bei welcher z. B. die Dämpfung und Phasenverschiebung einer elektromagnetischen Welle beim Durchtritt durch das Gemisch gemessen wird. In diesem Falle besteht der Sensor aus einem Sender und einem Empfänger. Entsprechende Techniken sind dem Fachmann bekannt.

Das zu messende Gemisch kann, wie bereits erwähnt, in flüssiger oder fester Form vorliegen. Es umfasst einen Trägerstoff (vorzugsweise flüssigen oder festen Beton), der den überwiegenden Volumenanteil des Gemisches bildet, sowie Wasser und Gas, welche z.B. in Poren oder Kavitäten im Trägerstoff angeordnet sind.

Zur Messung mindestens eines Parameters dieses Gemisches wird wie folgt vorgegangen.

In einem ersten Schritt wird das Gemisch 10 in den Messbereich des Sensors 2 bzw. 3 gebracht. Sodann wird dessen Temperatur T mit dem Temperatursensor 5 gemessen.

Nun wird mit dem Permittivitätswert-Messgerät 1 der komplexe Permittivitätswert $\varepsilon_m$ des sich im Messbereich 11 befindenden Gemisches ermittelt, und zwar bei mehreren Messfrequenzen $f_i$, vorzugsweise im Bereich zwischen 10 kHz und 10 GHz, vorzugsweise 10 MHz und 1 GHz.

Diese Messungen werden mit einer auf einem Modell eines Gemisches basierenden theoretischen Formel verglichen, vorzugsweise:

$$\varepsilon_m = \varepsilon_b + \sum_{i=1}^{n} \frac{v_i}{3} \cdot \left(\varepsilon_i - \varepsilon_b\right) \cdot \sum_{j=1}^{3} \frac{\varepsilon_m}{\varepsilon_m + N_{ij} \cdot \left(\varepsilon_i - \varepsilon_m\right)} \qquad (2)$$

Dabei bezeichnen $\varepsilon_1$ und $v_1$ Permittivitätswert und Volumenanteil des Gases, $\varepsilon_2$ und $v_2$ Permittivitätswert und Volumenanteil des freien Wassers, $\varepsilon_3$ und $v_3$ Permittivitätswert und Volumenanteil des gebundenen Wassers, $\varepsilon_b$ den Permittivitätswert des Trägerstoffes und $N_{1j}$, $N_{2j}$ und $N_{3j}$ die Depolarisierungsfaktoren einer ellipsoiden Kavität des Gases bzw. des freien Wassers bzw. des gebundenen Wassers. Werden die Einflüsse des gebundenen Wassers berücksichtigt, so ist $n = 3$. Werden diese Einflüsse vernachlässigt, so ist $n = 2$.

Durch Einsetzen der Messwerte $\varepsilon_m(f_i)$ in Gleichung (2) entsteht ein Gleichungssystem. Liegt eine ausreichende Zahl von Messwerten vor, so lassen sich durch Auswerten des Gleichungssystems verschiedene Parameter des Gemisches ermitteln, wie dies im folgenden beschrieben wird. Vorzugsweise wird dabei die Zahl der Messungen so gross gewählt, dass das Gleichungssystem überbestimmt ist und die Parameter durch Ausgleichsrechnung mit hoher Genauigkeit ermittelt werden können.

Dabei ist zu beachten, dass nebst Gleichung (2) weitere Ansätze und Näherungen existieren, die den Permittivitätswert $\varepsilon_m$ eines Gemisches abschätzen. Eine andere Gleichung, die in DE 196 52 679 beschrieben ist, ist die Mischungsformel von Polder-van Santen/de Loor. Weiter können z.B. für die Depolarisationsfaktoren verschiedene Näherungen gewählt werden. Beispielsweise können bei Annahme von Rotationssymmetrie für die Depolarisationsfaktoren des freien Wassers die folgende Näherungen verwendet werden:

$$N_{21} = N_{22} = N_{fw} \qquad (3)$$

$$N_{23} = 1 - 2 \cdot N_{fw},$$

d.h. die Depolarisationseffekte bei den Kavitäten des freien Wassers können durch einen einzigen Parameter $N_{fw}$ ausgedrückt werden.

Für die Depolarisationsfaktoren von Gas erweist sich folgende Annahme als sinnvoll:

$$N_{11} = N_{12} = N_{13} = 1/3 \qquad (4)$$

Für die Depolarisationsfaktoren des gebundenen Wassers kann folgende Näherung verwendet werden:

$$N_{31} = N_{32} = 0 \text{ und } N_{33} = 1. \tag{5}$$

Generell besitzen die Mischungsformeln bei Berücksichtigung des Einflusses des gebundenen Wassers die Form

$$\varepsilon_m = \varepsilon_m(\varepsilon_1, \varepsilon_2, \varepsilon_3, \varepsilon_b, v_1, v_2, v_3), \tag{6}$$

d.h. der Permittivitätswert des Gemisches wird als Funktion der Permittivitätswerte der Komponenten und der Volumenanteile angegeben. Gegebenenfalls können in Gleichung (6) noch weitere Parameter als unbekannte berücksichtigt werden, wie z.B. mindestens ein Depolarisationsfaktor einer Komponente des Gemisches, insbesondere ein Depolarisationsfaktor von freiem Wasser, z.B. der Parameter $N_{fw}$ aus Gleichung (3).

Wird der Einfluss des gebundenen Wassers nicht berücksichtigt bzw. vernachlässigt (oder näherungsweise als konstanter Beitrag zum Permittivitätswert $\varepsilon_b$ des Trägerstoffs berücksichtigt), und werden für die Depolarisationsfaktoren Näherungen der Art von Gleichung (3), (4) und (5) verwendet, so ergibt sich

$$\varepsilon_m = \varepsilon_m(\varepsilon_1, \varepsilon_2, \varepsilon_b, v_1, v_2, N_{fw}). \tag{7}$$

Einige der Parameter in Gleichungen (2), (6) oder (7) können mit ausreichender Genauigkeit abgeschätzt werden, während andere erst durch die Messung bestimmt werden können.

Der Permittivitätswert $\varepsilon_1$ des Gases kann bei den verwendeten Frequenzen in guter Näherung zu $1 + 0 \cdot i$ gesetzt werden.

Für den Permittivitätswert $\varepsilon_2$ des freien Wassers kann die Cole-Cole Näherung verwendet werden:

$$\varepsilon_2(f) = \varepsilon_{\infty(fw)} + \frac{\varepsilon_{stat(fw)} - \varepsilon_{\infty(fw)}}{1 + (i \cdot \omega \cdot \tau_{fw})^{1-\alpha}} - i \cdot \frac{\sigma_{fw}}{\omega \cdot \varepsilon_o}, \tag{8}$$

mit den Parametern $\varepsilon_{stat(fw)}$, $\varepsilon_{\infty(fw)}$), $\tau_{fw}$, $\alpha$, und $\sigma_{fw}$, wobei $\varepsilon_o = 8.8642 \times 10^{-12}$ F/m und $\omega = 2\pi f$. $\varepsilon_{stat(fw)}$ entspricht der statischen Dielektrizitätskonstante von freiem Wasser, $\varepsilon_{\infty(fw)}$) der Dielektrizitätskonstante von freiem Wasser bei optischen Frequenzen, $\tau_{fw}$ der Relaxationszeit von freiem Wasser, $\alpha = 0.02$ und $\sigma_{fw}$ der Leitfähigkeit von freiem Wasser. Numerische, temperatur- und salzabhängige Werte für die entsprechenden Grössen sind publiziert in "Permittivity Measurements Using Open-Ended Sensors and Reference Liquid Calibration - An Uncertainty Analysis", A. Nyshadham et al., IEEE Transactions on Microwave Theory and Techniques, Vol. 40(2), pp. 305ff, 1992.

Für den Permittivitätswert $\varepsilon_3$ des gebundenen Wassers kann ebenfalls die Cole-Cole Näherung verwendet werden:

$$\varepsilon_3(f) = \varepsilon_{\infty(bw)} + \frac{\varepsilon_{stat(bw)} - \varepsilon_{\infty(bw)}}{1 + (i \cdot \omega \, \tau_{bw})^{1-\alpha}} - i \cdot \frac{\sigma_{bw}}{\omega \cdot \varepsilon_o}, \tag{9}$$

mit den Parametern $\varepsilon_{stat(bw)}$, $\varepsilon_{\infty(bw)}$), $\tau_{bw}$, $\alpha$, und $\sigma_{bw}$ und mit $\varepsilon_o = 8.8642 \times 10^{-12}$ F/m und $\omega = 2\pi f$. Vorzugsweise werden folgende Grössen verwendet:

$\varepsilon_{stat(bw)} \approx 80$,
$\varepsilon_{\infty(bw)} \approx 4.5$,
$\tau_{bw} \approx -7.721 \times 10^{-14} T^3 + 1.017 \times 10^{-11} T^2 - 5.516 \times 10^{-10} T + 1.645 \times 10^{-8}$ Sekunden (Temperatur T in °C),
$\alpha = 0$, und
$\sigma_{bw} \approx 0$.

Der Permittivitätswert $\varepsilon_b$ des Trägerstoffs ist in der Regel aus Kalibriermessungen bekannt.

Die Volumenanteile $v_1$, $v_2$ und $v_3$ ergeben addiert die Porosität des Trägerstoffs. Wird der Einfluss des gebundene Wassers nicht berücksichtigt, so kann $v_3$ gleich Null gesetzt werden. In vielen praktischen Anwendungen ist der Volumenanteil $v_3$ eine feste Grösse, da das gebundene Wasser immer im Trägerstoff vorhanden und schwer zu entfernen ist. Für Beton besitzt $v_3$ einen Wert von etwa 0.016.

Aus Gleichung (7) (bzw. aus Gleichung (2) mit $v_3 = 0$ und mit den Näherungen (3) und (5)) ergibt sich beim Einsetzen von mindestens vier Messwerten bei unterschiedlicher Frequenz und unter Verwendung der oben erwähnten Werte für die bekannten Parameter ein Gleichungssystem, das die gleichzeitige Bestimmung folgender unbekannter Parameter erlaubt:

- Volumenanteil $v_1$ des Gases,
- Volumenanteil $v_2$ des freien Wassers,
- mindestens einen der Depolarisierungsfaktoren $N_{2j}$ des freien Wassers, insbesondere $N_{fw}$ bei Verwendung der Näherung (4), und
- elektrische Leitfähigkeit des freien Wassers.

Anstelle dieser Parameter können auch andere, von diesen Parametern abhängige Grössen bestimmt werden. Insbesondere kann z.B. aus der Leitfähigkeit des freien Wassers unter Verwendung von empirischen Gleichungen gemäss der oben erwähnten Veröffetlichung von A. Nyshadham et al. der Salzgehalt im freien Wasser ermittelt werden. Entsprechende Umrechnungsformeln sind dem Fachmann bekannt.

Wird der Einfluss des gebundenen Wassers nicht vernachlässigt und explizit berücksichtigt, so erhöht sich die Zahl der unbekannten Parameter. Es zeigt sich jedoch, dass bei guter Abschätzung des Permittivitätswerts $\varepsilon_3$ immer noch eine genaue Messung möglich ist. Hierzu ist es wichtig, dass berücksichtigt wird, dass dieser Permittivitätswert $\varepsilon_3$ bei den verwendeten Messfrequenzen frequenzabhängig ist, d.h. es gilt generell $\varepsilon_3 = \varepsilon_3(f)$. Als konkrete Formel kann z.B. Gleichung (9) eingesetzt werden. Je nach Frequenzbereich kann der Realwert von Gleichung (9) als konstante Grösse von z.B. 4.5 eingesetzt werden. Insbesondere gilt mit guter Näherung im genannten Frequenzbereich, wie bereits erwähnt, dass die Leitfähigkeit $\sigma_{bw}$ des gebundenen Wassers Null ist.

Somit kann auch unter Berücksichtigung des Einflusses des gebundenen Wassers mindestens ein Parameter, insbesondere der Volumenanteil $v_2$ des freien Wassers, aus Gleichung (2) bzw. (6) beim Einsetzen der Messwerte in das Gleichungssystem ermittelt werden.

Mit dem beschriebenen Verfahren kann auch die Porosität des Trägermaterials als Summe der Volumenanteile $v_1 + v_2$ (bzw. $v_1 + v_2 + v_3$ bei Berücksichtigung des gebundenen Wassers) ermittelt werden.

Weiter kann mit dem vorliegenden Verfahren die volumetrische Wassermenge aus dem Wert $v_2$ bzw. der Summe $v_2 + v_3$ bestimmt werden. Bei bekannter Reindichte des Trägermaterials, mit Einbeziehung der bekannten bzw. der bekannt gewordenen Porosität, kann vom volumenanteiligen auf den gewichtsanteiligen Wassergehalt wie folgt geschlossen werden:

$$\rho_{roh} = \rho_{rein} \cdot (1 - \theta)$$

mit:

$\rho_{roh}$     Rohdichte [z.B. g/cm$^3$]

$\rho_{rein}$     Reindichte [z.B. g/cm$^3$]

$\theta$     Porosität [ - ]

und:

$$w_{gew.} = \frac{w_{vol.}}{\rho_{roh}}$$

mit:

$W_{gew.}$     gewichtsbezogener Wassergehalt [M.-%]

$w_{vol.}$     volumenbezogener Wassergehalt [Vol. -%]

Die Reindichte kann auf einfache Weise im Labor durch Standardverfahren ermittelt werden.

In der obigen Diskussion wurde davon ausgegangen, dass der Permittivitätswert $\varepsilon_m$ des Gemisches ortsunabhängig ist. Trifft dies nicht zu, so ist der gemessene Wert $\varepsilon_m$ ein Mittelwert, d.h. ein integraler Wert, der Permittivität des Gemisches im Messbereich 11 des Sensors.

Insbesondere bei festen Gemischen ist der Permittivitätswert jedoch oftmals eine parametrisierte Funktion f der Tiefe x, d.h. des Abstandes von der Oberfläche, beispielsweise

$$f(x) = a1 + a2 \cdot (1\text{-}\exp(-x/a3)), \tag{9}$$

wobei $a1$, $a2$ und $a3$ unbekannte Parameter sind.

Es zeigt sich, dass das vorliegende Verfahren die Bestimmung des tiefenabhängigen Flüssigwasseranteils, oder analog eines anderen tiefenabhängigen Parameters (z.B. des Salzgehalts) erlaubt.

Hierzu werden mehrere Messschritte k durchgeführt, wobei in jedem Messschritt der Sensor in bekanntem Abstand vom Gemisch angeordnet und von diesem durch ein Dielektrikum mit bekannter Permittivität getrennt wird. Das Dielektrikum kann insbesondere auch Luft sein, und in einer der Messschritte beträgt der Abstand vorzugsweise 0. Zwischen den Messschritten wird der Abstand zwischen Sensor und Gemisch geändert, oder es wird ein anderes Dielektrikum zwischen Sensor und Gemisch eingeführt. In den meisten Messschritten wird der Messbereich 11 des Sensors somit nur teilweise und unterschiedlich stark in das Gemisch eindringen.

In jedem Messschritt wird mittels mehrerer Messungen bei unterschiedlichen Frequenzen ein vom integralen Permittivitätswert $\varepsilon_{mk}$ abhängiger Wert $w_k$ gemessen, wie z.B. der Wasseranteil oder der Salzgehalt. Hierzu kann z. B. davon ausgegangen werden, dass der Permittivitätswert $\varepsilon_m$ über den Messbereich konstant ist, so dass die oben beschriebenen Auswertungen verwendet werden können. Aus der unterschiedlichen Abhängigkeit der Werte $w_k$ vom zu bestimmenden Parameter (z.B. vom Feuchigkeitsanteil) im Messbereich können sodann z.B. durch Ausgleichs-rechnung die Grössen $a1$, $a2$, $a3$ und somit die Funktion $f$ bestimmt werden.

Vorzugsweise wird für jeden Messschritt $k$ das Integral

$$w_k = \int E_k(x) f_{a1, a2, \dots}(x)\, dx \tag{10}$$

berechnet, wobei $E_k(x)$ eine normierte Abhängigkeit der Empfindlichkeit des Sensors von der Tiefe $x$ im Gemisch bei dem im Messschritt $k$ herrschenden Bedingungen (Abstand zwischen Sensor und Gemisch und Permittivität des Dielektrikums) ist.

Die Abhängigkeit $E_k(x)$ kann z.B. durch vorhergehende Kalibrierungsmessungen unter den Messbedingungen des Messschrittes bestimmt oder numerisch, z.B. durch finite Elementberechnung abgeschätzt werden.

Beispielsweise kann von der Empfindlichkeit S(x) des auf dem Gemisch aufliegenden Sensors ausgegangen werden. Ist der Abstand zwischen Sensor und Gemisch im Messschritt $k$ gleich $d_k$, und ist der Permittivitätswert des Dielektrikums zwischen Sensor und Gemisch ungefähr gleich dem mittleren Permittivitätswert des Gemisches, so gilt näherungsweise

$$E_k(x) = S(x + d_k) \tag{11}$$

Durch Einsetzen der Messwerte $w_k$ in Gleichung (10) kann wiederum ein Gleichungssystem für die Parameter $a1$, $a2$, $a3$... aufgestellt werden, welches mittels Ausgleichsrechung lösbar ist.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

**Patentansprüche**

1. Verfahren zur Bestimmung mindestens eines Parameters eines Gemisches der Komponenten Trägerstoff, Wasser und Gas, umfassend folgende Schritte:

Einbringen des Gemisches in einen Messbereich eines Sensors
Messen komplexer Permittivitätswerte $\varepsilon_m(f_i)$ des Gemisches bei mehreren Messfrequenzen $f_i$ durch Beauf-schlagen des Sensors mit einer elektromagnetischen Welle und mit Hilfe von Kalibrierungsdaten des Sensors,
Aufstellen eines Gleichungssystems durch Einsetzen der Permittivitätswerte $\varepsilon_m(f_i)$ in eine Mischungsformel

$$\varepsilon_m \; = \; \varepsilon_b \; + \; \sum_{i=1}^{n} \frac{v_i}{3} \cdot \left( \varepsilon_i \; - \; \varepsilon_b \right) \cdot \sum_{j=1}^{3} \frac{\varepsilon_m}{\varepsilon_m \; + \; N_{ij} \cdot \left( \varepsilon_i \; - \; \varepsilon_m \right)}$$

mit $\varepsilon_1$ und $v_1$ Permittivitätswert und Volumenanteil des Gases, $\varepsilon_2$ und $v_2$ Permittivitätswert und Volumenanteil des freien Wassers, $\varepsilon_3$ und $v_3$ Permittivitätswert und Volumenanteil des gebundenen Wassers, $\varepsilon_b$ Permittivitätswert des Trägerstoffes und $N_{1j}$, $N_{2j}$ und $N_{3j}$ die Depolarisierungsfaktoren einer ellipsoiden Kavität des Gases bzw. des freien Wassers bzw. des gebundenen Wassers sind, wobei $n$ = 3 bei Berücksichtigung des gebundenen Wassers und n = 2 bei Vernachlässigung des gebundenen Wassers und
Ermitteln mindestens eines unbekannten Parameters der Mischungsformel oder eines aus dem unbekannten Parameter abgeleiteten Werts durch Auswertung des Gleichungssystems.

**2.** Verfahren nach Anspruch 1, **dadurch gekennzeichnet, dass** der Permittivitätswert $\varepsilon_3$ des gebundenen Wassers als frequenzabhängige Funktion $\varepsilon_3(f)$ in die Mischungsformel eingesetzt wird.

**3.** Verfahren nach Anspruch 1, wobei $n$ = 2, umfassend den folgenden Schritt:

Ermitteln der folgenden Parameter oder von den folgenden Parametern abhängiger Grössen aus dem Gleichungssystem:

- Volumenanteil $v_1$ des Gases,
- Volumenanteil $v_2$ des freien Wassers,
- den Depolarisierungsfaktoren $N_{fw}$ des freien Wassers und
- Leitfähigkeit des freien Wassers.

**4.** Verfahren nach einem der vorangehenden Ansprüche, wobei aus dem Gleichungssystem mindestens einer der Depolarisationskoeffizienten $N_{2j}$ für das freie Wasser bestimmt wird, und insbesondere wobei $N_{21} = N_{22} = N_{fw}$ und $N_{23} = 1 - 2 \cdot N_{fw}$ gesetzt und der Wert von $N_{fw}$ aus dem Gleichungssystem bestimmt wird.

**5.** Verfahren nach einem der vorangehenden Ansprüche, wobei für das gebundene Wasser $N_{31} = N_{32} = 0$ und $N_{33} = 1$ gesetzt wird.

**6.** Verfahren nach einem der vorangehenden Ansprüche, wobei für das Gas $N_{11} = N_{12} = N_{13} = 1/3$ gesetzt wird.

**7.** Verfahren nach einem der vorangehenden Ansprüche, **dadurch gekennzeichnet, dass** für den Permittivitätswert des gebundenen Wassers die folgende frequenzabhängige Funktion $\varepsilon_3(f)$ in die Mischungsformel eingesetzt wird

$$\varepsilon_3(f) = \varepsilon_{\infty(bw)} + \frac{\varepsilon_{stat(bw)} - \varepsilon_{\infty(bw)}}{1 + (i \cdot \omega \cdot \tau_{bw})^{1-\alpha}} - i \cdot \frac{\sigma_{bw}}{\omega \cdot \varepsilon_o},$$

mit den Parametern $\varepsilon_{stat(bw)}$, $\varepsilon_{\infty(bw)}$, $\tau_{bw}$, $\alpha$, und $\sigma_{bw}$ und mit $\varepsilon_o$ = 8.8642$\times 10^{-12}$ F/m und $\omega$ = 2$\pi f$, und insbesondere dass

$\varepsilon_{stat(bw)}$ ungefähr gleich 80, und/oder
$\varepsilon_{\infty(bw)}$) ungefähr gleich 4.5, und/oder
$\tau_{bw}$ abhängig von einer gemessenen Temperatur $T$ in Grad Celsius ungefähr gleich -7.721$\times 10^{-14} T^3$ + 1.017$\times 10^{-11} T^2$ - 5.516$\times 10^{-10} T$ + 1.645$\times 10^{-8}$ Sekunden und/oder
$\alpha$ gleich Null und/oder
$\sigma_{bw}$ gleich Null gesetzt wird.

**8.** Verfahren nach einem der vorangehenden Ansprüche, wobei der Volumenanteil $v_3$ des gebundenen Wassers als konstante Grösse in das Gleichungssystem eingesetzt wird, und insbesondere dass $v_3$ für Beton ungefähr gleich 0.016 gesetzt wird.

**9.** Verfahren nach einem der vorangehenden Ansprüche, **dadurch gekennzeichnet, dass** für den Permittivitätswert des freien Wassers die folgende frequenzabhängige Funktion $\varepsilon_2(f)$ in die Mischungsformel eingesetzt wird

$$\varepsilon_2(f) = \varepsilon_{\infty(fw)} + \frac{\varepsilon_{stat(fw)} - \varepsilon_{\infty(fw)}}{1 + (i \cdot \omega \cdot \tau_{fw})^{1-\alpha}} - i \cdot \frac{\sigma_{fw}}{\omega \cdot \varepsilon_o},$$

mit den Parametern $\varepsilon_{stat(fw)}$, $\varepsilon_{\infty(fw)}$, $\tau_{fw}$, $\alpha$, und $\sigma_{fw}$ und mit $\varepsilon_o = 8.8642 \times 10^{-12}$ F/m und $\omega = 2\pi f$, wobei $\varepsilon_{stat(fw)}$ der statischen Dielektrizitätskonstante von freiem Wasser, $\varepsilon_{\infty(fw)}$) der Dielektrizitätskonstante von freiem Wasser bei optischen Frequenzen, $\tau_{fw}$ einer Relaxationszeit von freiem Wasser und $\sigma_{fw}$ einer Leitfähigkeit von freiem Wasser entspricht.

10. Verfahren nach Anspruch 9, wobei die Leitfähigkeit $\sigma_{fw}$ des freien Wassers ermittelt wird.

11. Verfahren nach einem der vorangehenden Ansprüche, wobei aus einer Abhängigkeit des Imaginärteils der Permittivität des freien Wassers von der Leitfähigkeit $\sigma_{fw}$ des freien Wassers aus dem Gleichungssystem eine Salzkonzentration im freien Wasser ermittelt wird.

12. Verfahren nach einem der vorangehenden Ansprüche, wobei der Trägerstoff ein Feststoff mit Poren ist, und wobei das Gas und das Wasser in den Poren sind.

13. Verfahren nach Anspruch 12, wobei der Trägerstoff Beton ist.

14. Verfahren nach einem der vorangehenden Ansprüche, wobei die Temperatur des Gemisches gemessen wird.

15. Verfahren nach einem der vorangehenden Ansprüche, wobei der Sensor als Oberflächensensor auf eine glatte, ebene Oberfläche des Trägerstoffs aufgesetzt wird oder der Trägerstoff in flüssiger Form in den Sensor eingefüllt wird.

16. Verfahren nach einem der vorangehenden Ansprüche, wobei bei mindestens drei unterschiedlichen Messfrequenzen gemessen wird.

17. Verfahren nach einem der vorangehenden Ansprüche, wobei aus der Summe der Volumenanteile eine Porosität des Trägerstoffs ermittelt wird.

18. Verfahren nach einem der vorangehenden Ansprüche, **dadurch gekennzeichnet, dass** die Messfrequenzen im Bereich von 10 kHz und 10 GHz, vorzugsweise von 10 MHz bis 1 GHz liegen.

19. Verfahren nach einem der vorangehenden Ansprüche, wobei aus dem Gleichungssystem der Volumenanteil $v_2$ des freien Wassers ermittelt wird.

20. Verfahren nach einem der vorangehenden Ansprüche zum Bestimmen eines tiefenabhängigen Parameters, insbesondere des Wasseranteils, eines Gemisches mit den Komponenten Trägerstoff, Wasser und Gas, umfassend mehrere Messschritte k, wobei mindestens in einem Teil der Messschritte ein Sensor in bekanntem Abstand vom Gemisch angeordnet und von diesem durch ein Dielektrikum mit bekannter Permittivität getrennt wird, derart, dass ein Messbereich des Sensors in unterschiedlichen Messschritten unterschiedlich stark ins Gemisch eindringt, wobei mit dem Sensor eine vom integralen Permittivitätswert $\varepsilon_{mk}$ des Gemisches im Messbereich abhängiger Wert $w_k$ gemessen wird, und eine Auswertung, in welcher über eine Abhängigkeit der Werte $w_k$ vom zu bestimmenden Parameter ein tiefenabhängiger Verlauf des Flüssigwasseranteils ermittelt wird.

21. Verfahren nach Anspruch 20, wobei bei der Auswertung ein parametrisierter Verlauf des Parameters an die Werte $w_k$ angepasst wird.

22. Verfahren nach einem der Ansprüche 20 oder 21, wobei zwischen den Messschritten der Abstand zwischen dem Sensor und dem Gemisch geändert wird und/oder unterschiedliche Dielektrika zwischen dem Sensor und dem Gemisch angeordnet werden.

23. Verfahren nach einem der Ansprüche 20 bis 22, wobei der effektive Permittivitätswert unter Annahme eines über den Messbereich konstanten zu messenden Parameters ermittelt wird.

**24.** Verfahren nach einem der Ansprüche 20 bis 23, wobei für jeden Messschritt k das Integral

$$w_k = \int E_k(x) f_{a1,a2,\dots}(x)\, dx,$$

berechnet wird, wobei $E_k$(x) eine normierte Abhängigkeit einer Empfindlichkeit des Sensors von der Tiefe *x* im Gemisch bei gegebenem Abstand zwischen Sensor und Gemisch und Permittivität des Dielektrikums in Messschritt k ist, und $f_{a1,a2,a3,\dots}$(x) eine mit Parametern *a*1, *a*2, *a*3 ... behaftete erwartete tiefenabhängige Verteilung des Parameters im Gemisch ist,
und wobei die Parameter *a*1, *a*2, *a*3 ... aus den Integralen der Messschritte durch Ausgleichsrechnung ermittelt werden.

**25.** Vorrichtung zur Durchführung des Verfahrens nach einem der vorangehenden Ansprüche, wobei sie einen Sensor (2, 3) ein Messgerät (1) zur frequenzabhängigen Ausmessung des Sensors und eine Datenverabeitungsvorrichtung (4) aufweist, **dadurch gekennzeichnet, dass**, die Datenverabeitungsvorrichtung (4) ausgestaltet ist, um den zu bestimmenden Parameter nach einem der vorangehenden Ansprüche zu ermitteln.

**26.** Vorrichtung nach Anspruch 25, wobei der Sensor (2) ein Oberflächensensor (2) oder ein Hohlleitersensor (3) ist.

**27.** Vorrichtung nach einem der Ansprüche 25 oder 26, wobei zwischen Sensor (2, 3) und Messgerät (1) eine Koaxialleitung (14) angeordnet ist und der Sensor (2, 3) einen sich verjüngenden Übergangsteil (12) zum impedanzangepassten Anschluss an die Koaxialleitung (14) aufweist.

**28.** Vorrichtung nach einem der Ansprüche 25 bis 27, wobei das Messgerät (1) einen vektoriellen Netzwerkanalysator aufweist, und ein vom Netzwerkanalysator ermittelter Reflexions- und/oder Transmissionsfaktor mit Hilfe sensorspezifischer Kalibrierungsdaten in eine Permittivität des Gemisches umwandelbar ist.

**Claims**

**1.** Method for determining at least one parameter of a mixture of the components of a carrier substance, water and gas, comprising the following steps:

bringing the mixture into a measuring range of a sensor,
measuring complex permittivity values $\varepsilon_m(f_i)$ of the mixture at several measuring frequencies $f_i$ by feeding an electromagnetic wave to the sensor and by means of calibration data of the sensor,
establishing a system of equations by entering the permittivity values $\varepsilon_m(f_i)$ into a mixing formula

$$\varepsilon_m = \varepsilon_b + \sum_{i=1}^{n} \frac{v_i}{3} \cdot (\varepsilon_i - \varepsilon_b) \cdot \sum_{j=1}^{3} \frac{\varepsilon_m}{\varepsilon_m + N_{ij} \cdot (\varepsilon_i - \varepsilon_m)}$$

with $\varepsilon_1$ and $v_1$ being the permittivity value and the volume fraction of the gas, $\varepsilon_2$ and $v_2$ the permittivity value and the volume fraction of the free water, $\varepsilon_3$ and $v_3$ the permittivity value and the volume fraction of the bound water, $\varepsilon_b$ the permittivity value of the carrier substance, and $N_{1j}$, $N_{2j}$ and $N_{3j}$ the depolarisation factors of an ellipsoidal cavity of the gas or the free water or the bound water, respectively, wherein $n = 3$ when taking the bound water into account and $n = 2$ when neglecting the bound water, and
determining at least one unknown parameter of the mixing formula, or of a value derived from the unknown parameter, by evaluating the system of equations.

**2.** Method according to claim 1, **characterised in that** the permittivity value $\varepsilon_3$ of the bound water is inserted as a frequency dependent function $\varepsilon_3$(f) into the mixing formula.

**3.** Method according to claim 1, wherein n=2, comprising the following step:

determining the following parameters, or values depending on the following parameters, from the system of equations:

- volume fraction $v_1$ of the gas,
- volume fraction $v_2$ of the free water,
- the depolarisation factors $N_{fw}$ of the free water and
- conductivity of the free water.

4. Method according to any of the preceding claims, wherein at least one of the depolarisation factors $N_{2j}$ of the free water is determined from the system of equations, and in particular that $N_{21} = N_{22} = N_{fw}$ and $N_{23} = 1 - 2 \cdot N_{fw}$ is used and the value of $N_{fw}$ is determined from the system of equations.

5. Method according to any of the preceding claims, wherein for the bound water $N_{31} = N_{32} = 0$ and $N_{33} = 1$ is used.

6. Method according to any of the preceding claims, wherein for the gas $N_{11} = N_{12} = N_{13} = 1/3$ is used.

7. Method according to any of the preceding claims, **characterised in that** for the permittivity value of the bound water the following frequency dependent function $\varepsilon_3(f)$ is inserted into the mixing formula:

$$\varepsilon_3(f) = \varepsilon_{\infty(bw)} + \frac{\varepsilon_{stat(bw)} - \varepsilon_{\infty(bw)}}{1 + (i \cdot \omega \cdot \tau_{bw})^{1-\alpha}} - i \cdot \frac{\sigma_{bw}}{\omega \cdot \varepsilon_o},$$

with the parameters $\varepsilon_{stat(bw)}$, $\varepsilon_{\infty(bw)}$), $\tau_{bw}$, $\alpha$, and $\sigma_{bw}$ and with $\varepsilon_o = 8.8642 \times 10^{-12}$ F/m and $\omega = 2\pi f$, and in particular that

$\varepsilon_{stat(bw)}$ is approximately equal to 80 and/or
$\varepsilon_{\infty(bw)}$) is approximately equal to 4.5 and/or
$\tau_{bw}$ is, depending on a measured temperature $T$ in degree Celsius, approximately equal to $-7.721 \times 10^{-14} T^3 + 1.017 \times 10^{-11} T^2 - 5.516 \times 10^{-10} T + 1.645 \times 10^{-8}$ seconds, and/or
$\alpha$ is set to zero and/or
$\sigma_{bw}$ is set to zero.

8. Method according to any of the preceding claims, wherein the volume fraction $v_3$ of the bound water is inserted as a constant quantity into the system of equations and in particular that $v_3$ is set approximately equal to 0.016 for concrete.

9. Method according to any of the preceding claims, **characterised in that** the following frequency dependent function $\varepsilon_2(f)$ is inserted into the mixing formula for the permittivity value of the free water:

$$\varepsilon_2(f) = \varepsilon_{\infty(fw)} + \frac{\varepsilon_{stat(fw)} - \varepsilon_{\infty(fw)}}{1 + (i \cdot \omega \cdot \tau_{fw})^{1-\alpha}} - i \cdot \frac{\sigma_{fw}}{\omega \cdot \varepsilon_o},$$

with the parameters $\varepsilon_{stat(fw)}$, $\varepsilon_{\infty(fw)}$, $\tau_{fw}$, $\alpha$, and $\sigma_{fw}$ and with $\varepsilon_O = 8.8642 \times 10^{-12}$ F/m and $\omega = 2\pi f$, wherein $\varepsilon_{stat(fw)}$ corresponds to the static dielectric constant of free water, $\varepsilon_{\infty(fw)}$ to the dielectric constant of free water at optical frequencies, $\tau_{fw}$ to a relaxation time of free water and $\sigma_{fw}$ to a conductivity of free water.

10. Method according to claim 9, wherein the conductivity $\sigma_{fw}$ of the free water is determined.

11. Method according to any of the preceding claims, wherein using the system of equations a salt concentration in the free water is determined from a dependence of the imaginary part of the permittivity of the free water from the conductivity $\sigma_{fw}$ of the free water.

12. Method according to any of the preceding claims, wherein the carrier substance is a solid with pores and wherein the gas and the water are in the pores.

13. Method according to claim 12, wherein the carrier substance is concrete.

14. Method according to any of the preceding claims, wherein the temperature of the mixture is measured.

15. Method according to any of the preceding claims, wherein the sensor is placed as a surface sensor onto a smooth, flat surface of the carrier substance or the carrier substance is filled into the sensor in liquid form.

16. Method according to any of the preceding claims, wherein measurements are made at at least three different frequencies.

17. Method according to any of the preceding claims, wherein a porosity of the carrier substance is determined from the sum of the volume fractions.

18. Method according to any of the preceding claims, **characterised in that** the measuring frequencies are in a range of 10 kHz and 10 GHz, in particular of 10 MHz to 1 GHz.

19. Method according to any of the preceding claims, wherein the volume fraction $v_2$ of the free water is determined from the system of equations.

20. Method according to any of the preceding claims for determining a depth dependent parameter, in particular the amount of water, of a mixture with the components carrier substance, water and gas, comprising
    several measuring steps $k$, wherein at least in a part of the measuring steps a sensor is arranged at a known distance from the mixture and is separated from the same by a dielectric of known permittivity, such that a measuring range of the sensor is extending to different depths into the mixture in the different measuring steps, wherein, by means of the sensor, a value $w_k$ depending on an integral permittivity value $\varepsilon_{mk}$ of the mixture in the measuring range is measured, and
    an evaluation in which a depth dependence of the liquid water fraction is determined based on a dependency of the values $w_k$ from the parameter to be determined.

21. Method according to claim 20, wherein in the evaluation a parametric function of the parameter is fitted to the values $w_k$.

22. Method according to any of the claims 20 or 21, wherein, between the measuring steps, the distance between the sensor and the mixture is changed and/or different dielectrics are arranged between the sensor and the mixture.

23. Method according to any of the claims 20 to 22, wherein the effective permittivity value is determined under the assumption that the parameter to be measured is constant over the measuring range.

24. Method according to any of the claims 20 to 23, wherein for each measuring step k the integral

$$w_k = \int E_k(x) f_{a1,a2,\ldots}(x)\,dx,$$

    is calculated, wherein $E_k(x)$ is a normalised dependence of a sensitivity of the sensor from the depth $x$ in the mixture at a given distance between the sensor and the mixture and permittivity of the dielectric in the measuring step $k$, and $f_{a1,a2,a3,\ldots}(x)$ is an expected depth dependent distribution of the parameter in the mixture with parameters $a1$, $a2$, $a3\ldots$,
    wherein the parameters $a1$, $a2$, $a3\ldots$ are determined from the integrals of the measuring steps by means of calculus of observations.

25. Device for carrying out the method of any of the preceding claims, wherein it comprises a sensor (2, 3), a measuring apparatus (1) for a frequency dependent measurement of the sensor, and a data processing apparatus (4), **characterised in that** the data processing apparatus (4) is designed for determining the parameter to be determined according to any of the preceding claims.

26. Device according to claim 25, wherein the sensor (2) is a surface sensor (2) or a waveguide sensor (3).

27. Device according to any of the claims 25 or 26, wherein a coaxial transmission line (14) is arranged between the sensor (2, 3) and the measuring apparatus (1) and the sensor (2, 3) comprises a tapered transition section (12) for an impedance matched connection to the coaxial transmission line (14).

28. Device according to any of the claims 25 to 27, wherein the measuring apparatus (1) comprises a vector network analyser, and a reflection and/or transmission factor determined by the network analyser can be converted into a permittivity of the mixture by means of sensor specific calibration data.

**Revendications**

1. Procédé de détermination d'au moins un paramètre d'un mélange des composants englobant un support, de l'eau et un gaz, comprenant les étapes suivantes :

   - introduction du mélange dans une zone de mesure d'un capteur;
   - mesure de valeurs de permittivité $\varepsilon_m(f_i)$ complexes du mélange en présence de plusieurs fréquences de mesure $f_i$, par application d'une onde électromagnétique au capteur et à l'aide de données d'étalonnage du capteur;
   - établissement d'un système d'équations par insertion des valeurs de permittivité $\varepsilon_m(f_i)$ dans une formule du mélange

$$\varepsilon_m = \varepsilon_b + \sum_{i=1}^{n} \frac{v_i}{3} \cdot (\varepsilon_i - \varepsilon_b) \cdot \sum_{j=1}^{3} \frac{\varepsilon_m}{\varepsilon_m + N_{ij} \cdot (\varepsilon_t - \varepsilon_m)}$$

   - $\varepsilon_1$ et $v_1$ étant la valeur de permittivité et la part de volume du gaz, $\varepsilon_2$ et $v_2$ étant la valeur de permittivité et la part de volume de l'eau libre, $\varepsilon_3$ et $v_3$ étant la valeur de permittivité et la part de volume de l'eau combinée, $\varepsilon_b$ étant la valeur de permittivité du support et $N_{1j}$, $N_{2j}$ et $N_{3j}$ étant les coefficients de dépolarisation d'une cavité ellipsoïdale du gaz ou de l'eau libre ou de l'eau combinée, avec n = 3 en cas de prise en compte de l'eau combinée et n = 2 en cas de non prise en compte de l'eau combinée, et
   - détermination d'au moins un paramètre inconnu de la formule du mélange ou d'une valeur dérivée de ce paramètre inconnu, par résolution du système d'équations.

2. Procédé selon la revendication 1, **caractérisé en ce que** la valeur de permittivité $\varepsilon_3$ de l'eau combinée est insérée dans la formule du mélange en tant que fonction dépendant de la fréquence $\varepsilon_3(f)$.

3. Procédé selon la revendication 1, avec n = 2, comprenant l'étape suivante: détermination des paramètres suivants ou de grandeurs dépendant des paramètres suivants, à partir du système d'équations:

   - part de volume $v_1$ du gaz,
   - part de volume $v_2$ de l'eau libre,
   - coefficients de dépolarisation $N_{fw}$ de l'eau libre, et
   - conductivité de l'eau libre.

4. Procédé selon une des revendications précédentes, selon lequel on détermine à partir du système d'équations au moins un des coefficients de dépolarisation $N_{2j}$ pour l'eau libre, en définissant notamment $N_{21} = N_{22} = N_{fw}$ et $N_{23} = 1-2 * N_{fw}$ et en déterminant la valeur de $N_{fw}$ à partir du système d'équations.

5. Procédé selon une des revendications précédentes, selon lequel on définit $N_{31} = N_{32} = 0$ et $N_{33} = 1$ pour l'eau combinée.

6. Procédé selon une des revendications précédentes, selon lequel on définit $N_{11} = N_{12} = N_{13} = 1/3$ pour le gaz.

7. Procédé selon une des revendications précédentes, **caractérisé en ce que** la fonction dépendant de la fréquence $\varepsilon_3(f)$ suivante est insérée dans la formule du mélange pour la valeur de permittivité de l'eau combinée:

$$\varepsilon_3(f) = \varepsilon_{\infty(bw)} + \frac{\varepsilon_{stat(bw)} - \varepsilon_{\infty(bw)}}{1 + (i \cdot \omega \cdot \tau_{bw})^{1-\alpha}} - i \cdot \frac{\sigma_{bw}}{\omega \cdot \varepsilon_o},$$

avec les paramètres $\varepsilon_{stat(bw)}$, $\varepsilon_{\infty(bw)}$, $\tau_{bw}$, $\alpha$ et $\sigma_{bw}$ et avec $\varepsilon_o = 8.8642 \times 10^{-12}$ F/m et $\omega = 2\pi f$, et notamment **en ce que**

   - $\varepsilon_{stat(bw)}$ est défini à peu près égal à 80, et/ou

- $\varepsilon_{\infty(bw)}$ est défini à peu près égal à 4.5, et/ou
- $\tau_{bw}$, en fonction d'une température T mesurée en degrés Celsius, est défini à peu près égal à $-7.721\times10^{-14}T^3 + 1.017\times10^{-11}T^2 - 5.516\times10^{-10}T + 1.645\times10^{-8}$ secondes et/ou
- $\alpha$ est défini égal à zéro et/ou
- $\sigma_{bw}$ est défini égal à zéro.

8. Procédé selon une des revendications précédentes, selon lequel la part de volume $v_3$ de l'eau combinée est insérée en tant que grandeur constante dans le système d'équations et notamment $v_3$ pour le béton est défini à peu près égal à 0.016.

9. Procédé selon une des revendications précédentes, **caractérisé en ce que** la fonction dépendant de la fréquence $\varepsilon_2(f)$ suivante est insérée dans la formule du mélange pour la valeur de permittivité de l'eau libre:

$$\varepsilon_2(f) = \varepsilon_{\infty(fw)} + \frac{\varepsilon_{stat(fw)}\ \varepsilon_{\infty(fw)}}{1+(i\cdot\omega\cdot\tau_{fw})^{1-\alpha}} - i\cdot\frac{\sigma_{fbw}}{\omega\cdot\varepsilon_o},$$

avec les paramètres $\varepsilon_{stat(fw)}$, $\varepsilon_{\infty(fw)}$, $\tau_{fw}$, $\alpha$ et $\sigma_{fw}$ et avec $\varepsilon_o = 8.8642\times10^{-12}$ F/m et $\omega = 2\pi f$, $\varepsilon_{stat(fw)}$ correspondant à la constante diélectrique statique de l'eau libre, $\varepsilon_{\infty(fw)}$ à la constante diélectrique de l'eau libre pour des fréquences optiques, $\tau_{fw}$ à un temps de relâchement de l'eau libre et $\sigma_{fw}$ à une conductivité de l'eau libre.

10. Procédé selon la revendication 9, selon lequel on détermine la conductivité $\sigma_{fw}$ de l'eau libre.

11. Procédé selon une des revendications précédentes, selon lequel on détermine, à l'aide du système d'équations, une concentration de sel dans l'eau libre à partir d'une dépendance de la partie imaginaire de la permittivité de l'eau libre vis-à-vis de la conductivité $\sigma_{fw}$ de l'eau libre.

12. Procédé selon une des revendications précédentes, selon lequel le support est une matière solide avec des pores et le gaz et l'eau se trouvent dans les pores.

13. Procédé selon la revendication 12, selon lequel le support est du béton.

14. Procédé selon une des revendications précédentes, selon lequel on mesure la température du mélange.

15. Procédé selon une des revendications précédentes, selon lequel le capteur est posé en tant que capteur de surface sur une surface plane et lisse du support ou le support est introduit sous une forme liquide dans le capteur.

16. Procédé selon une des revendications précédentes, selon lequel la mesure est réalisée avec au moins trois fréquences de mesure différentes.

17. Procédé selon une des revendications précédentes, selon lequel on détermine une porosité du support à partir de la somme des parts de volume.

18. Procédé selon une des revendications précédentes, **caractérisé en ce que** les fréquences de mesure sont comprises dans la plage allant de 10 kHz à 10 GHz, de préférence de 10 MHz à 1 GHz.

19. Procédé selon une des revendications précédentes, selon lequel on détermine la part de volume $v_2$ de l'eau libre à partir du système d'équations.

20. Procédé selon une des revendications précédentes pour déterminer un paramètre dépendant de la profondeur, notamment la part d'eau, d'un mélange des composants englobant un support, de l'eau et un gaz, comprenant :

- plusieurs étapes de mesure k, un capteur étant disposé, au moins pour une partie des étapes de mesure, à une distance connue du mélange et étant séparé de celui-ci par un diélectrique de permittivité connue, de telle sorte qu'une zone de mesure du capteur, à des étapes de mesure différentes, pénètre plus ou moins profondément dans le mélange, le capteur servant à mesurer une valeur $w_k$ qui dépend de la valeur de permittivité intégrale $\varepsilon_{mk}$ du mélange dans la zone de mesure, et
- un dépouillement, lors duquel on détermine une variation de la part d'eau liquide en fonction de la profondeur, par l'intermédiaire d'une dépendance des valeurs $w_k$ vis-à-vis du paramètre à déterminer.

21. Procédé selon la revendication 20, selon lequel, au cours du dépouillement, une évolution paramétrée du paramètre est adaptée aux valeurs $w_k$.

22. Procédé selon une des revendications 20 ou 21, selon lequel on modifie la distance entre le capteur et le mélange, entre les étapes de mesure, et/ou on dispose des diélectriques différents entre le capteur et le mélange.

23. Procédé selon une des revendications 20 à 22, selon lequel la valeur de permittivité effective est déterminée dans l'hypothèse d'un paramètre à mesurer constant sur la plage de mesure.

24. Procédé selon une des revendications 20 à 23, selon lequel on calcule pour chaque étape de mesure k l'intégrale

$$w_k = \int E_k(x) f_{a1,a2,\ldots}(x) dx,$$

$E_k(x)$ désignant une dépendance normalisée d'une sensibilité du capteur vis-à-vis de la profondeur x dans le mélange, avec une distance donnée entre le capteur et le mélange et une permittivité donnée du diélectrique à l'étape de mesure k, et $f_{a1,\,a2,\,a3,\ldots}(x)$ étant une répartition attendue, comportant les paramètres a1, a2, a3 ..., du paramètre dans le mélange, en fonction de la profondeur,
et les paramètres a1, a2, a3 ... étant déterminés à partir des intégrales des étapes de mesure, par calcul de compensation.

25. Dispositif pour la mise en oeuvre du procédé selon une des revendications précédentes, qui présente un capteur (2, 3), un appareil de mesure (1) pour effectuer la mesure du capteur en fonction de la fréquence et un moyen de traitement de données (4), **caractérisé en ce que** le moyen de traitement de données (4) est conçu pour déterminer le paramètre à définir, conformément à l'une des revendications précédentes.

26. Dispositif selon la revendication 25, dans lequel le capteur (2) est un capteur de surface (2) ou un capteur à guide d'ondes (3).

27. Dispositif selon une des revendications 25 ou 26, dans lequel une ligne coaxiale (14) est disposée entre le capteur (2, 3) et l'appareil de mesure (1) et le capteur (2, 3) et le capteur (2, 3) présente une partie de transition (12) rétrécie, servant à la connexion à la ligne coaxiale (14), avec adaptation de l'impédance.

28. Dispositif selon une des revendications 25 à 27, dans lequel l'appareil de mesure (1) présente un analyseur de réseau vectoriel, et un coefficient de réflexion et/ou de transmission déterminé par l'analyseur de réseau peut être converti en une permittivité du mélange à l'aide de données d'étalonnage spécifiques au capteur.

**Fig. 1**

**Fig. 2**

**Fig. 3**